(19) Europäisches Patentamt
European Patent Office
Office européen des brevets

(11) Publication number: 0 058 890 B1

# (12) EUROPEAN PATENT SPECIFICATION

(45) Date of publication of patent specification: 26.09.84

(51) Int. Cl.[3]: C 07 D 233/60, A 61 K 31/415

(21) Application number: 82101023.8

(22) Date of filing: 11.02.82

## (54) Antithrombotic compounds.

(30) Priority: 12.02.81 GB 8104402

(43) Date of publication of application: 01.09.82 Bulletin 82/35

(45) Publication of the grant of the patent: 26.09.84 Bulletin 84/39

(84) Designated Contracting States: BE CH DE FR GB LI LU NL SE

(56) References cited:
US-A-3 707 475
US-A-3 708 598
US-A-3 772 441

(73) Proprietor: THE WELLCOME FOUNDATION LIMITED
183-193 Euston Road
London NW1 2BP (GB)

(72) Inventor: Phillips, Arthur Page
1400 Kershaw Drive
Raleigh North Carolina 27609 (US)
Inventor: White, Helen Lyng
210 Ridgecrest Drive
Chapel Hill North Carolina 27514 (US)
Inventor: Rosen, Solomon
260 East 25th Street Apartment 5D
New York, N.Y. 10010 (US)

(74) Representative: Berg, Wilhelm, Dr. et al
Dr. Berg, Dipl.-Ing. Stapf, Dipl.-Ing. Schwabe, Dr. Dr. Sandmair Mauerkircherstrasse 45
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 058 890 B1

## Description

This invention relates to compounds useful in medicine, to the preparation of such compounds, to pharmaceutical formulations containing such compounds and the preparation of such formulations, and to the use of the compounds in medicine.

Platelets, the smallest formed elements of the blood, play an important role in hemostasis by stopping the outward flow of blood through ruptures in the blood vessel wall. They aggregate or fuse together at the site of injury to form a temporary plug or clot which seals the blood vessel. The clumping together of the platelets initiates a chain of biochemical reactions which leads to the reinforcing of the clot with the fibrous protein, fibrin.

Aggregation of platelets appears to be triggered by their contact with certain surfaces, such as the basement membranes of the blood vessels or collagen fibrils. Once aggregation has begun, the platelets stick to each other.

It is clear that the ability of platelets to aggregate and initiate thrombus formation is important to protect the organism from excessive loss of blood as a result of injury. However, in some individuals, platelets will begin to adhere to the inner walls of the blood vessels as a result of a disease process. As more platelets accumulate, a blood clot may form which restricts the amount of blood flowing through the vessel. This can result in the interruption of blood flow to a vital organ such as the heart, for example, thereby causing a heart attack. A thrombus formed in this way may also become dislodged and travel as an embolus through the circulatory system. In such a case, there is a risk that the embolus could become lodged in vessels supplying organs such as the brain or lung and could restrict the flow of blood so much that the function of these organs would be severely impaired. This could be fatal or render the individual disabled.

Further general discussions of blood platelets are presented by S. B. Kahn, *American Family Physician, Vol. 21* No. 5, 166, (1980) and M. B. Zucker, *Scientific American Vol. 242*, p 86, June (1980) and *Vol. 204*, p 58, February (1961).

It is clear from the foregoing discussion that it is desirable under some situations to reduce or prevent the aggregation of blood platelets *in vivo* in order to avoid the pathological disturbances to the cardiovascular system noted above.

It is an object of the present invention to provide a class of compounds which have been found to possess an advantageous inhibitory effect upon the aggregation of blood platelets. These compounds have, in addition, been found to possess useful anti-inflammatory, anti-pyretic and analgesic activities, as discussed in more detail below.

A number of substituted triphenyl-imidazoles is described in the US—A—3 708 598. An antithrombotic effect of these substances is not mentioned in this document.

In particular, the present invention relates to compounds of Formula (I),

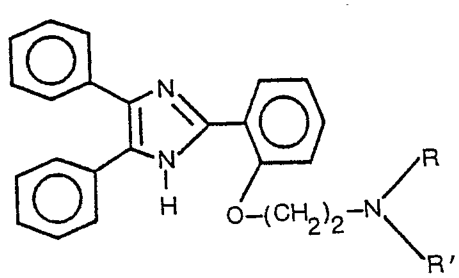

(I)

in which R and R′ are identical or different and may be hydrogen; a lower alkyl having 1 to 4 carbons, eg., methyl or ethyl; phenyl; phenyl substituted with one or more (eg., 1 or 2) lower alkyl groups having from 1 to 4 carbon atoms, eg., methyl or ethyl, lower alkoxy groups having from 1 to 4 carbons, eg., methoxy or ethoxy, or halogen atoms, e.g. Br or Cl; phenyl-$C_{1-4}$-alkyl (optionally substituted on the phenyl ring with one or more (e.g., 1 or 2) lower alkyl groups having from 1 to 4 carbons, eg., methyl or ethyl, lower alkoxy groups having 1 to 4 carbons, eg., methoxy or ethoxy or halogen atoms, e.g. Br or Cl); or taken together with adjacent nitrogen atom form a cyclic substituent of 5 or 6 members containing in addition to the nitrogen atom an optional second hetero atom selected from the group consisting of oxygen, nitrogen and sulfur. Any reference hereinafter to the compounds of Formula (I) may be taken to include reference to the acid addition salts and solvates including hydrates thereof. The therapeutic activities of these compounds reside in the base so that useful acid addition salts may be prepared from various acids which are pharmaceutically acceptable. Representative acid salts include but by no means are limited to hydrochloride (mono and di), hydrogensulfate (mono and di), isothionate (mono and di), sulfate, maleate, mesylate (mono and di), diphosphate, and ditartrate.

The preferred embodiment of this invention relates to compounds of Formula (I) in which R and R′ are identical or different and may be hydrogen, a lower alkyl having 1 or 2 carbons, phenylmethyl (benzyl), or phenylethyl (optionally substituted on the phenyl ring with one or two methoxy, methyl or

chloro groups) or taken together with the adjacent nitrogen atom form a cyclic substituent of 5 or 6 members containing in addition to the nitrogen atom an optional second hetero atom which is oxygen or nitrogen.

Of the preferred embodiment, the following compounds have been found to be particularly useful:

2-[2-(2-Aminoethoxy)phenyl]-4,5-diphenylimidazole (Ia)

2-{2-[2-(Methylamino)ethoxy]phenyl}-4,5-diphenylimidazole (Ib)

2{2-[2-(Diethylamino)ethoxy]phenyl}-4,5-diphenylimidazole (Ic)

2-{2-[2-(*N*-Methylbenzylamino)ethoxy]phenyl}-4,5-diphenylimidazole (Id)

2-{2-[2-(4-Methoxy-*N*-methylbenzylamino)ethoxy]phenyl}-4,5-diphenylimidazole (Ie)

2-{2-[2-(3,4-Dimethoxy-*N*-methylbenzylamino)ethoxy]phenyl}-4,5-diphenylimidazole (If)

2-{2-[2-(*N*,4-dimethylbenzylamino)ethoxy]phenyl}-4,5-diphenylimidazole (Ig)

2-{2-[2-(4-Chloro-*N*-methylbenzylamino)ethoxy]phenyl}-4,5-diphenylimidazole (Ih)

4,5-Diphenyl-2-{2-[2-(*N*-propylbenzylamino)ethoxy]phenyl}imidazole (Ii)

2-{2-[2-(Dibenzylamino)ethoxy]phenyl}-4,5-diphenylimidazole (Ij)

2-{2-[2-(*N*-Methylphenethylamino)ethoxy]phenyl}-4,5-diphenylimidazole (Ik)

4,5-Diphenyl-2-[2-(2-piperidinoethoxy)phenyl]imidazole (Il)

2-[2-(2-Morpholinoethoxy)phenyl]-4,5-diphenylimidazole (Im)

4,5-Diphenyl-2-{2-[2-(1-pyrrolidinyl)ethoxy]phenyl}imidazole (In)

The most preferred embodiment of this invention is the compound of Formula (II) (including its pharmaceutically acceptable acid addition salts such as hydrochloride, sulfate, phosphate, isothionate, and solvates including the hydrate) also known as 2-{2-[2-(dimethylamino)ethoxy]phenyl}-4,5-diphenylimidazole hereinafter referred to as the "Compound II."

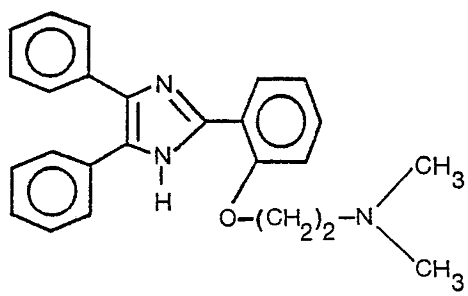

(II)

The compounds of Formula (I) and their physiologically acceptable acid addition salts and solvates are useful for the treatment or prophylaxis of thrombo-embolic conditions in a mammal, such conditions including those whose etiology is associated with platelet aggregation. The compounds of Formula (I) have been found, when it is desired to inhibit platelet aggregation, to prevent the formation of thrombi in mammals, including man. For example these compounds would be useful in prevention of myocardial infarctions, transient ischemic attacks (TIA's), strokes, post-operative thrombosis (including that incurred after artificial heart valve or hip replacement), central retinal vein occlusions and the atherosclerotic process that leads to a variety of conditions including peripheral vascular diseases and renal vascular hypertension. Other treatable conditions include diabetic retinopathy, thrombotic thrombocytopenia purpura and migraine. Compounds of Formula (I) would be useful in maintaining A—V shunt patency in uremic patients maintained on hemodialysis.

They may also be used as additives to blood, blood products, blood substitutes, and other fluids which are used in artificial extra-corporeal circulation and perfusion of isolated body portions, e.g., limbs and organs, whether attached to the original body, detached and being preserved or prepared for transplant, or attached to a new body. During these circulations and perfusion, aggregated platelets

tend to block the blood vessels and portions of the circulation apparatus. This blocking may be avoided by the presence of the compounds of Formula (I).

The compounds of Formula (I), especially the compound II, are also useful as substitutes for nonsteroidal drugs in treatment of fever, acute inflammation, and rheumatoid arthritis. One important advantage of the above compounds is that they have been found to be relatively devoid of gastric effects shown in animal models. Compound II is useful as a substitute for aspirin®, indomethacin, and other non-steroidal anti-inflammatory agents in the treatment of acute inflammation and fever.

Another important advantage of the compounds of Formula (I) is the high solubility of their di-acid salts. This is a very useful property since it allows these compounds to be used in an injectable form.

The compounds of Formula (I) may be used in the relief, treatment or prophylaxis of pain, inflammation and/or fever in a mammal, including man, such as: that resulting from headache, toothache, pain following general dental procedures, oral and general surgery, dysmenorrhea, myalgia, pain of unresectable cancer, joint and peripheral nerve disorders, rheumatoid arthritis, rheumatoid spondylitis, osteo-arthritis, gouty arthritis, pyresis and other conditions associated with pain, inflammation and/or fever.

The amount of active compound required for use in the above conditions will, of course, vary both with the route of administration, the condition under treatment and the mammal undergoing treatment, and is ultimately at the discretion of the physician. However, a suitable antithrombotic, analgesic, anti-inflammatory and/or anti-pyretic oral dose of the active compound for a mammal is in the range of from 1 to 100 mg per kilogram body weight per day; a typical dose of, e.g., Compound II for a human recipient being 30 mg/kg body weight per day.

The desired dose is preferably presented as from one to three sub-doses administered at appropriate intervals throughout the day. Thus, where two sub-doses of Compound II are employed each will preferably lie in the range of from 0.5 to 30 mg/kg body weight; a typical subdose for a human recipient being about 10 mg/kg body weight.

While it is possible for the active compound to be administered (at an appropriate dose) alone, it is preferable to present it as a pharmaceutical formulation. Formulations of the present invention, both for veterinary and for human medical use, comprise the active compound together with one or more pharmaceutically acceptable excipients therefor and optionally any other desired therapeutic ingredient. The excipient(s) or carrier(s) must be pharmaceutically acceptable in the sense of being compatible with the other ingredients of the formulation and not deleterious to the recipient thereof. The other therapeutic ingredient(s) may include: caffeine; other analgesics, anti-inflammatories or anti-pyretics such as aspirin or codeine; antitussives, expectorants; bronchodilators; or anti-histamines.

The formulation of the present invention include those suitable for oral, rectal or parenteral (including subcutaneous, intraperitoneal, intramuscular and intravenous) administration.

The formulations may be adapted to provide for rapid, prolonged or delayed release of the active compound to effect different rates of onset and/or duration of therapeutic effect.

The formulations may conveniently be presented in unit dosage form and may be prepared by any of the methods well known in the art of pharmacy. All methods include the step of bringing the active compound into association with one or more excipient(s). In general, the formulations are prepared by uniformly and intimately bringing the active compound into association with a liquid, a semi-solid, a bulk solid or a polymeric material, or a combination thereof, and then forming the product into desired formulation.

Formulations of the present invention suitable for oral administration may be presented as discrete units such as capsules (soft or hard gelatin), cachets, tablets (coated or uncoated), lozenges or boluses, each containing a predetermined amount of the active compound; or as solutions or suspensions of the active compound for effective treatment in a aqueous liquid, or a combination thereof, such as: a syrup, an elixir, a draught, a solution, a suspension, a linctus or a drench; or as an oil-in-water emulsion, a water-in-oil emulsion or a multiple emulsion; a paste or a gel.

Compounds of Formula (I) and their salts may be prepared by any method known in the art for the preparation of compounds of analogous structure. For the present-invention, the following processes are provided wherein "R" and "R′" have the same meaning as for compounds of Formula (I) herein before.

1) The reaction of a $\alpha,\beta$-diphenylethylene derivative with an appropriate 2(2-aminoethyloxy)phenyl derivative, eg. an aldehyde amidine, nitrile etc., to form the imidazole ring produces compounds of Formula (I). The ethylene derivative may be $\alpha,\beta$-diketone, an $\alpha$-ketone with a leaving group in the $\beta$ position or an $\alpha$-amino-ketone. With some reactions of this type, an ammonium derivative may be required as a source of nitrogen. Preferable embodiments of this method are:

a) A compound of Formula (III) may be reacted with benzil and ammonium acetate.

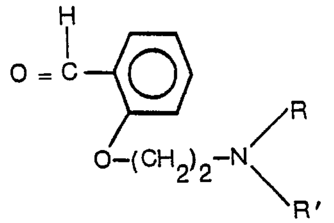

(III)

This condensation may be effected in such a solvent such as acetic acid at reflux temperature. The compounds of Formula (III) may be prepared by reacting a compound of formula HNRR′ with the compound of Formula (IV).

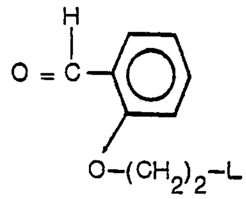

(IV)

where L is a suitable leaving group as defined by J. March, *Advanced Organic Chemistry* 3rd ed page *187* (1977), McGraw Hill, NY, eg. L = Halogen (such as bromine or chlorine) or other leaving group such as toluenesulfonate.

In turn, the compound of Formula (IV) wherein L is halogen may be prepared by the reaction of salicylaldehyde and 1,2-dihaloethane in an alkanol such as methanol at reflux temperature in the presence of a base such as sodium hydroxide.

b) A compound of Formula (V) where "G" is a suitable leaving group such as halide, hydroxy or toluenesulfonate, may be reacted with a compound of Formula (VI) in a suitable solvent with or without a catalyst, e.g., *acid* or *base*, present.

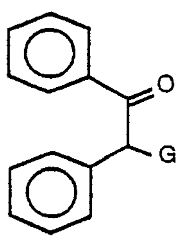

(V)

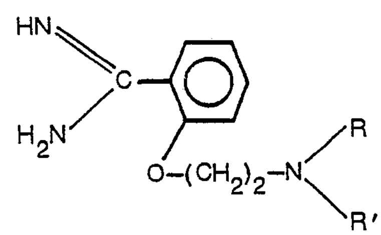

(VI)

Compounds of Formula (VI) can be prepared from compounds of formula (VIa) by such a method as that described first by Pinner and Klein, *Ber, 10,* 1889 (1877), and more recently by Roger and Neilson, *Chem. Rev., 61* 179 (1961).

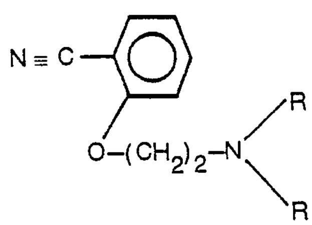

(VIa)

In turn compounds of Formula (VIa) may be prepared from 2-hydroxy benzonitrile by a similar procedure described herein above for the preparation of Formula (III).

c) A compound of Formula (VII) may be reacted with a compound of Formula (VIII) in a suitable solvent.

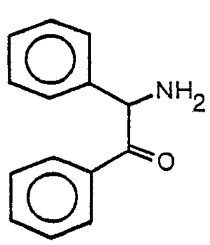

(VII)

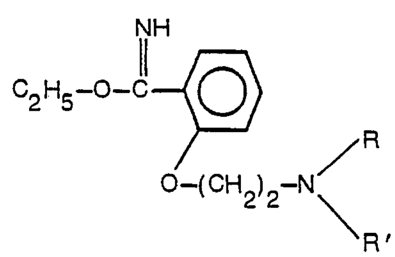

(VIII)

A compound of Formula (VIII) may be prepared from compounds of Formula VIa described before herein by such a method as that described by Pinner, *Die Imidoether Und ihre Perivate,* (Berlin, 1892), reprinted Ann Arbor, Mich., 1961, page 23. For a review of imino esters, see Neilson, in Patai, "The Chemistry of Amidines and Imidates," pp. 385—489, John Wiley & Sons, New York, 1975.

d) A compound of formula (VII) described in method C herein above may be reacted with a compound of Formula (IX).

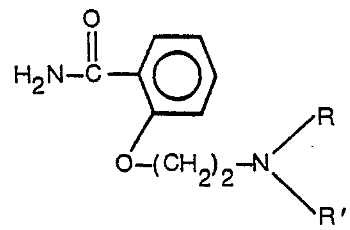

(IX)

The compounds of Formula (IX) can be prepared from 2-hydroxy benzamide by an analogous route to that for the compounds of Formula (III) described herein above.

2) Another method comprises a reaction of the compound of Formula (X) with a compound of Formula (XI) in the presence of a base in a suitable solvent.

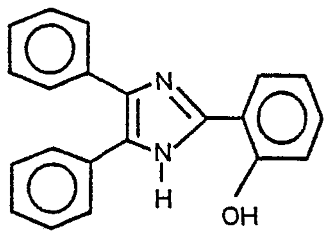

(X)

wherein L is a leaving group such as that in Formula (IV).

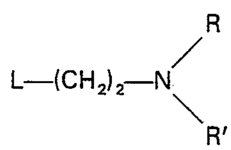

(XI)

The compound (X) can be prepared by condensation of benzil, ammonium acetate and salicylaldehyde in an analogous way to that described in method 1a.

3) A compound of Formula (XII) may be reacted with an appropriate amine, HNRR, in a suitable solvent preferably with a non-nucleophilic base present such as $K_2CO_3$.

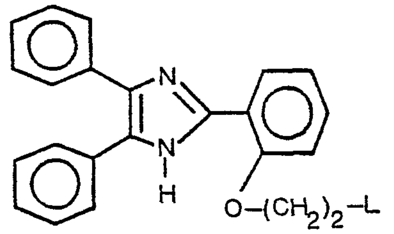

(XII)

$L-(CH_2)_2-L'$ (XIII)

The compound of Formula (XII) can be made from reacting the compound of Formula (X) with a compound of Formula (XIII), wherein L and L′ are suitable leaving groups (such as those described in relations to L in Formula (IV) above, in a suitable solvent in the presence of a base.

4) The reaction of a compound of Formula I (wherein at least one of R and R′ is hydrogen) with an appropriate alkylating, arylating or aralkylating agent, to provide a corresponding compound of Formula (I) (wherein at least one of R and R′ is a lower alkyl, phenyl or phenyl-$C_{1-4}$-alkyl group as defined above).

5) A further method comprises the condensation of the compound of Formula (XIV) with a primary or secondary amine, HNRR′ in the presence of a reducing agent such as hydrogen or sodium borohydride.

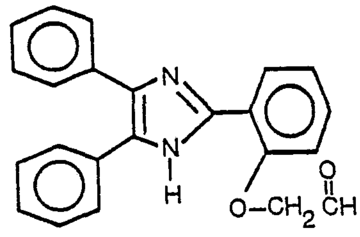

(XIV)

The compound of Formula (XIV) can be prepared by known methods of mild oxidation of compound formula (XV).

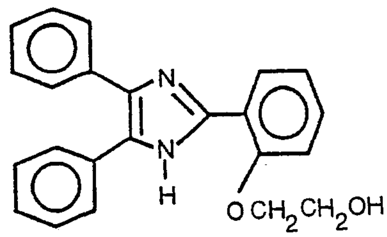

(XV)

The following Examples illustrate the present invention

Example 1

Preparation of 2-{2-[2-(Dimethylamino)ethoxy]phenyl}-4,5-diphenylimidazole (Compound II)

a. 2-(2-Bromoethoxy)benzaldehyde

Salicylaldehyde (620 g, 5.08 mole), 1,2-dibromoethane (3814 g 20.3 mole) and methanol (5.0 liters) were heated to reflux, then with stirring, 6N sodium hydroxide (1.0 liter) was added in 100 mL portions with 30 minute intervals between additions. After 18 hours of stirring at reflux, the methanol was removed under reduced pressure (aspirator). Water (1.5 liters) was added to the resulting slurry and the mixture was extracted with diethyl ether (2.5 liters). The organic phase was separated and filtered through CELITE® brand filter aid to remove the solid material. The filtrate was then washed with 6N sodium hydroxide (3 x 250 mL) followed by water (3 x 500 mL).

The ether layer was separated, dried over anhydrous $K_2CO_3$, filtered and stripped of solvent on the steam bath. The resulting residue was crystallized from a 1:1 diethyl ether-pentane mixture, air dried and 2-(2-bromoethoxy)benzaldehyde, 565 g (48.5% theoretical yield), was obtained. An analytical sample was obtained by column chromatography on silica gel using toluene as eluent, m.p. 58—60.5°C.

| | | | |
|---|---|---|---|
| Elemental Analysis: | Calcd. for $C_9H_9O_2Br$ | | MW 229.08 |
| | C 47.19; | H 3.96 | |
| Found: | C 47.21; | H 3.92 | |

b. 2-(2-Dimethylaminoethoxy)benzaldehyde

Dimethylamine (25% aqueous solution, 1.2 liters), 2-(2-bromoethoxy)benzaldehyde (229 g, 1.0 mole) and methanol (4.0 liters) were stirred at ambient temperature for 60 hours. The reaction mixture was stripped of solvent at aspirator pressure on a steam bath to yield a light yellow residue. This residue was dissolved in 4N NaOH (500 mL) and extracted with three, 800 mL portions of ether. The ether extracts were combined, extracted with, 4N hydrochloric acid (4 x 125 mL) followed by washing with water (125 mL). The hydrochloric acid and water washes were combined and made alkaline with of 8N NaOH (250 mL), then extracted with diethyl ether (3 x 800 mL). The ether extracts were combined and washed with water (3 x 250 mL). The organic phase was dried with $K_2CO_3$, filtered and stripped of solvent to yield the crude product.

The crude material was distilled at high vacuum with 151 g (78.2% of theoretical yield) of 2-(2-dimethylaminoethoxy)benzaldehyde being collected at 101°C and 0.30 mm Hg.

| | | | |
|---|---|---|---|
| Elemental Analysis: | Calcd. for $C_{11}H_{15}NO_2$ | | MW 193.25 |
| | C 68.37; | H 7.82; | N 7.25 |
| Found: | C 68.13; | H 7.92; | N 7.14 |

c. 2-{2-[2-(Dimethylamino)ethoxy]phenyl}4,5-diphenylimidazole

Benzil (223 g, 1.06 mole), ammonium acetate (340 g, 4.41 mole), 2-(2-dimethylaminoethoxy)-benzaldehyde (228 g, 1.18 mole) and glacial acetic acid (700 mL) were refluxed for two hours then poured into ice and water (about 7 liters). Concentrated ammonium hydroxide (about 3 liters) was added in small amounts to make the mixture basic. The solid material was collected and recrystallized from methanol to yield 2-{2-[2-(dimethylamino)ethoxy]phenyl}4,5-diphenylimidazole, 297 g (73% of theoretical yield), m.p. 146.5—148.5°C.

| | | | |
|---|---|---|---|
| Elemental Analysis: | Calcd. for $C_{25}H_{25}N_3O$ | | MW 383.47 |
| | C, 78.30; | H, 6.57; | N, 10.96. |
| Found: | C, 78.22; | H, 6.59; | N, 10.94 |

Example 2

Preparation of 2-{2-[2-(*N*-methylbenzylamino)ethoxy]phenyl}4,5-diphenylimidazole (Id)

a. 2-(2-*N*-Methylbenzylaminoethoxy)benzaldehyde

Methylbenzylamine (26.6 g, 0.22 mole), 2-(2-bromoethoxy)benzaldehyde (23.0 g, 0.1 mole) and methanol (50 mL) were heated on a steam bath with stirring for 4 hr. The solvent was stripped at aspirator pressure on a steam bath to leave a red-brown liquid which was stirred with diethyl ether (500 mL) and then the methylbenzylamine hydrobromide was removed by filtration. The ether solution was extracted with 24% NaOH solution (2 x 25 mL) then extracted with 3N HCl (4 x 25 mL). The acidic solution was made basic with 24% NaOH solution and then extracted with ether (4 x 100 mL). All ether extracts were combined and dried over $K_2CO_3$, filtered and the solvent removed to give 26.3 g of red liquid.

Water (50 mL) and acetic anhydride (50 mL) were added to the red liquid and stirred for an hour to convert the unreacted methylbenzylamine amine into the amide. Ether (~300 mL) was added and the resulting mixture was extracted with 3N HCl (4 x 25 mL). The acid solution was made basic with NaOH (24% solution) and this alkaline solution was extracted with ether (4 x 152 mL). All the ether extracts were combined and dried over anhydrous $K_2CO_3$. After filtering, removal of solvent yielded 25.8 g (96% of theoretical yield) of 2-(2-*N*-methylbenzylaminoethoxy)benzaldehyde as a red-brown liquid.

| | | | |
|---|---|---|---|
| Elemental Analysis: | Calcd. for $C_{17}H_{19}NO_2$ | | MW 269.3 |
| | C 75.81; | H 7.11; | N 5.20 |
| Found: | C 75.57; | H 7.16; | N 5.32 |

b. 2-{2-[2-(*N*-Methylbenzylamino)ethoxy]phenyl}4,5-diphenylimidazole

Benzil (16 g, 0.076 mole), ammonium acetate (23.1 g, 0.3 mole), 2-(2-methylbenzylaminoethoxy)benzaldehyde (20.5 g, 0.076 mole) and glacial acetic acid (120 mL) were refluxed for four hours then poured into of ice and water (about 500 mL). Concentrated ammonium hydroxide (28%) (about 225 mL) was added to make the mixture basic and the solid precipitate was collected and recrystallized from ethylacetate to yield 2-{2-[2-(methylbenzylamino)ethoxy]phenyl}-4,5-diphenylimidazole 16.6 g (47% of theoretical yield), mp. 206—207°C.

| | | | |
|---|---|---|---|
| Elemental Analysis: | Calcd. for $C_{31}H_{29}N_3O$ | | MW 459.57 |
| | C 81.01; | H 6.36; | N 9.15 |
| Found: | C 81.12; | H 6.35; | N 9.15 |

Example 3

Preparation of 4,5-Diphenyl-2-[2-(2-piperidinoethoxy)phenyl]imidazole (II)

a. 2-(2-Piperidinoethoxy)benzaldehyde

The preparation was analogous to that described in example 2 except 1 molar equivalent of bromoethoxybenzaldehyde and 2.3 to 3 equivalents of piperidine were heated at reflux in methanol for three hours. The methanol was evaporated and the residue was worked-up as in example 1b. The 2-(2-piperidinoethoxy)benzaldehyde produced this way was used in its crude state in the below preparation, 3b.

b. 4,5-Diphenyl-2-[2-(2-piperidinoethoxy)phenyl]imidazole

Benzil, ammonium acetate and 2-(2-piperidinoethoxy)benzaldehyde were reacted in the same molar proportions and under the same conditions as in the analogous preparation disclosed in example 1c. The yield of 2{2-[2-(piperidino)ethoxy]phenyl}-4,5-diphenylimidazole after recrystallization from methanol was 75% with a mp of 184—185°C.

| | | | |
|---|---|---|---|
| Elemental Analysis: | Calcd. for $C_{28}H_{29}N_3O$ | | MW 423.53 |
| | C, 79.40; | H, 6.90; | N, 9.92 |
| Found: | C, 79.34; | H, 6.89; | N, 9.93 |

Example 4

Preparation of 2-[2-(2-Morpholinoethoxy)phenyl]-4,5-diphenylimidazole (Im)

a. 2-(2-Morpholinoethoxy)benzaldehyde

The preparation of 2-(2-morpholinoethoxy)benzaldehyde was the same as described in example 3a, except an appropriate amount of morpholine was used in place of piperidine.

b. 2-[2-(2-Morpholinoethoxy)phenyl]-4,5-diphenylimidazole

The same procedure was used as described in example 3b, except 2-(2-morpholinoethoxy)-benzaldehyde was used in place of 2-(2-piperidinoethoxy)benzaldehyde. The yield of 2{2-[2-(morpholino)ethoxy]phenyl}4,5-diphenylimidazole was 60%, and the mp was 173—174°C.

| | | | |
|---|---|---|---|
| Elemental Analysis: | Calcd. for $C_{27}H_{27}N_3O_2$ | | MW 425.54 |
| | C, 76.21; | H, 6.40; | N, 9.87 |
| Found: | C, 76.72; | H, 6.40; | N, 9.77 |

Example 4a

Preparation of 2-(2-(2-Anilinoethoxy)phenyl]-4,5-diphenylimidazole

The preparation is analogous to that described in Example 2 except 1 molar equivalent of bromoethoxybenzaldehyde 2.5 to 3 molar equivalents of aniline are heated at reflux in methanol for three hours. The methanol is evaporated and the residue is reacted as in example 1b. To produce 2-[2-(2-Anilinoethoxy)phenyl]-4,5-diphenylimidazole.

Example 5

Preparation of Acid Addition Salts of Compound II

a. Preparation of 2-{2-[2-(Dimethylamino)ethoxy]phenyl}-4,5-diphenylimidazole sulfate, i.e. Compound II sulfate

12 g of Compound II was dissolved in 180 mL of methanol and 2.0 mL of sulfuric acid was slowly added. The product crystallized upon cooling and was collected, washed with ether and dried to give 10.3 g (64.7%) of Compound II sulfate; m.p. 145.5—148°C.

| | | | |
|---|---|---|---|
| Elemental Analysis: | Calcd. for $C_{25}H_{25}N_3O{\cdot}H_2SO_4{\cdot}1\frac{1}{2}H_2O$ | | |
| | C, 59.04; | H, 5.95; | N, 8.26 |
| Found: | C, 58.99; | H, 5.99; | N, 8.23 |

b. Preparation of Compound II maleate

2 g of Compound II was dissolved in 14 mL of toluene and 0.67 g of maleic acid dissolved in 5 mL acetone was added. The mixture was stirred vigorously. The product crystallized and was collected, washed with ether and dried to give 2.6 g (100%) of Compound II maleate; mp 131—133.5°C.

| | | | |
|---|---|---|---|
| Elemental Analysis: | $C_{25}H_{25}N_3O{\cdot}C_4H_4O_4$ | | |
| | C, 69.72; | H, 5.85; | N, 8.41 |
| Found: | C, 69.55; | H, 5.92; | N, 8.37 |

c. Preparation of Compound II dihydrochloride

250 g of Compound II was dissolved in 4 L of methanol at 55°. The solution was treated with 54 g of hydrogen chloride and 25 g Darco G—60® and stirred at reflux. The carbon was removed by filtration and the filtrate was concentrated to a 1 L volume, 2.5 L of ethyl acetate was added and the mixture was cooled. The product was collected, washed with ethyl acetate and dried to give 287 g (96.5%) of Compound II dihydrochloride, mp 277—280°C.

| | | | | |
|---|---|---|---|---|
| Elemental Analysis: | Calcd. for $C_{25}H_{25}N_3O{\cdot}2HCl$ | | | |
| | C, 65.79; | H, 5.96; | N, 9.21; | Cl, 15.53 |
| Found: | C, 65.70; | H, 5.97; | N, 9.19; | Cl, 15.48 |

d. Preparation of Compound II hydrochloride (as monohydrate)

a. 15.4 g of Compound II was dissolved in a mixture of 75 mL methanol, 60 mL water and 9 mL hydrochloric acid. The solution was treated with 2*N* NaOH, adjusting to pH 5.14, causing precipitation of the product. The product was collected, washed with water, slurried in acetone, recollected and dried to give 11.6 g (66%) of Compound II hydrochloride.

| | | | | |
|---|---|---|---|---|
| Elemental Analysis: | Calcd. for $C_{25}H_{25}H_3O{\cdot}HCl{\cdot}H_2O$ | | | |
| | C, 68.56; | H, 6.44; | N, 9.59; | Cl, 8.09 |
| Found: | C, 68.38; | H, 6.49; | N, 9.55; | Cl, 8.05 |

e. Preparation of Compound II hydrochloride (as dihydrate)

Compound II (1811.8 g, 4.73 mole), methanol (2,463 mL) and 1*N* hydrochloric acid (4,724.6 mL, 4.72 mole) were combined and heated to 60°C. The mixture was cooled to −5°C and the solids filtered. The white solids were vacuum dried at 50°C giving 1,791 g on an anhydrous basis (90% of theoretical yield).

| | | | | | |
|---|---|---|---|---|---|
| Elemental Analysis: | Calcd. for $C_{25}H_{25}N_3O{\cdot}HCl{\cdot}2.03\ H_2O$ | | | | MW 456.52 |
| | C, 65.77; | H, 6.64; | N, 9.20; | Cl, 7.77 | |
| Found: | C, 65.77; | H, 6.62; | N, 9.20; | Cl, 7.79 | |

f. Preparation of Compound II diisothionate and isethionate

16 g of Compound II was dissolved in a solution of 7.56 g isethionic acid in methanol-water. The solution was concentrated to an oil which was redissolved in ethanol. The solution was diluted with acetone to crystallize Compound II diisothionate which was collected, washed and dried giving 10.0 g (47.1%); mp 113—115°C.

The filtrate and wash were concentrated to a powder which was triturated with ethanol-acetone, collected and dried to give 5.6 g (26.4%) of Compound II isothionate; mp 87—89°C.

| | | | | |
|---|---|---|---|---|
| Elemental Analyses: | Calcd. for $C_{25}H_{25}N_3O{\cdot}2C_2H_6O_4S{\cdot}2\frac{1}{2}H_2O$ | | | |
| | C, 51.16; | H, 6.22; | N, 6.17; | S, 9.42 |
| Found: | C, 51.19; | H, 6.24; | N, 6.10; | S, 9.43 |
| | Calcd. for $C_{25}H_{25}N_3O{\cdot}C_2H_6O_4S{\cdot}2H_2O$ | | | |
| | C, 59.43; | H, 6.47; | N, 7.70; | S, 5.88 |
| Found: | C, 59.45; | H, 6.50; | N, 7.66; | S, 5.87 |

g. Acid addition salts of other compounds of Formula (I) may be prepared in a manner analogous to that described above.

Example 6

Preparation of Other Compounds of Formula (I)

a. The compounds listed in Table I were prepared in an analogous way to that described in Example 1.

TABLE I

| Compound | Compound Number | Melting Point (°C) |
|---|---|---|
| 2-[2-(2-Aminoethoxy)phenyl]-4,5-diphenylimidazole | Ia | 139—140 |
| 2-{2-[2-(Methylamino)ethoxy]phenyl}-4,5-diphenylimidazole | Ib | 135—136 |
| 2-{2-[2-(Diethylamino)ethoxy]phenyl}-4,5-diphenylimidazole | Ic | 79— 80 |

b. The compounds listed in Table II were prepared in an analogous way to that described in Example 2

TABLE II

| Compound | Compound Number | Melting Point (°C) |
|---|---|---|
| 2-{2-[2-(4-Methoxy-*N*-methylbenzylamino)-ethoxy]phenyl}-4,5-diphenylimidazole | Ie | 210—212[1] |
| 2-{2-[2-(3,4-Dimethoxy-*N*-methylbenzylamino)-ethoxy]phenyl}-4,5-diphenylimidazole | If | 247—249[1] |
| 2-{2-[2-(*N*,4-Dimethylbenzylamino)ethoxy]-phenyl}-4,5-diphenylimidazole | Ig | 185—186[1] |
| 2-{2-[2-(4-Chloro-*N*-methylbenzylamino)ethoxy]-phenyl}-4,5-diphenylimidazole | Ih | 223—225[1] |
| 4,5-Diphenyl-2-(2-[2-(*N*-propylbenzylamino)-ethoxy]phenyl)imidazole | Ii | 173—175[1] |
| 2-{2-[2-(Dibenzylamino)ethoxy]phenyl}-4,5-diphenylimidazole | Ij | 123—125 |
| 2-{2-[2-(*N*-Methylphenylamino)ethoxy]phenyl}-4,5-diphenylimidazole | Ik | 111—112 |

[1]Dihydrochloride salt prepared from the base as described in Example 5 above.

c. 4,5-Diphenyl-2-{2-[2-(1-pyrrolidinyl)ethoxy]phenyl}imidazole (In), mp 154—155°C, was prepared in an analogous way to that described in Examples 3 and 4.

Example 7
Inhibition of Platelet Aggregation

a. *In vitro studies:* Concentrations of 2 $\mu$M or less partially inhibit, while 4 $\mu$M completely inhibits arachidonate and collagen-induced aggregation of platelets from human, guinea pig, rat, and dog. In these tests, described by Born[1], Compound II is at least 25-fold more potent than aspirin and equipotent to indomethacin.

b. *Ex vivo studies:* The aggregation of platelets taken from animals dosed by the oral route with Compound II is inhibited when tested with arachidonate and collagen by the method of Born ((1962) Nature *194,* 927, 929). The dose required to inhibit aggregation rate by 50% ($ED_{50}$) at 1 hour was 5 mg/kg and 10 mg/kg in the guinea pig and rat, respectively. By comparison, the $ED_{50}$ for aspirin in the guinea pig was 30 mg/kg, while the $ED_{50}$ for aspirin in the rat was 10 mg/kg. The $ED_{50}$ for indomethacin was 10 mg/kg and 5 mg/kg in the guinea pig and rat, respectively. In the dog, 10 mg/kg p.o. was an $ED_{50}$ for as long as 6 hr. The second (release) phase of ADP-induced aggregation of dog platelets was also inhibited by 50% at 6 hours after 10 mg/kg p.o.

Like indomethacin but unlike aspirin, the inhibitions of aggregation by Compound II in the rat and guinea pig is reversible such that platelets taken 24 hours after dosing respond normally to aggregation stimuli.

Example 8
Anti-thrombotic activity *in vivo*

a. *Prevention of formalin-induced carotid artery thrombosis:* Topical application of formalin to a carotid artery of the guinea pig produces a local, occlusive, platelet-dependent thrombus within 5 hours (Beek, O. G. and Abrahams, S. L. Fed. Proc. *39,* 423 (1980)). Twenty-five mg/kg p.o. of Compound II (administered 1 hr. prior and again at 2 hr. post-formalin application) protected 50% of the animals from thrombosis. The corresponding $ED_{50}$'s for aspirin and indomethacin were 2 × 35 mg/kg p.o. and 2 × 50 mg/kg p.o., respectively.

b. *Protection against stasis-induced jugular vein thrombosis:* When a 1 cm length of guinea pig jugular vein is ligated *in situ*, a thrombus forms within 30 min. Six mg/kg p.o. of either Compound II or aspirin given 1 hour prior to ligation protected 50% of the animals from thrombosis.

Example 9

Anti-Inflammatory Activity

a. *Carrageenin Pleurisy Assay (Acute):* Following the procedure described by Vinegar *et al. (Eur. J. Rheumatology and Inflammation, 1,* 204 (1978)), Compound II is slightly less potent by the oral route than aspirin in the carrageenin pleurisy assay in the rat. In this assay, the 3 hr. $ED_{50}$ is 37 mg/kg for Compound II vs. 28 mg/kg and 2 mg/kg, respectively, for aspirin and indomethacin against exudate fluid volume.

b. *Adjuvant Arthritis Assay (Chronic):* When administered by gavage for 14 days, Compound II inhibits the development of adjuvant arthritis in the rat as determined by the method of Vinegar *et al. (J. Immunopharmacology, 1,* 497 (1979)). The $ED_{50}$ was 294 mg/kg/day compared with 175 and 1 mg/kg/day for aspirin and indomethacin, respectively.

Example 10

Mild Analgesic Activity

a. Acetic Acid Writhing Test

Using the procedure described by Koster *et al. (Fed. Proc., 18,* 412 (1959)) and Vinegar *et al. (Handbook of Experimental Pharmacology, 50—52,* ch. 26, *Anti-Inflammatory Drugs,* Ed. Vane, J. and Ferreria, S. (1978)), the acetic acid writhing test was performed, using the rat, to demonstrate the mild analgesic activity of the compound of Formula (II).

Compound II is approximately 2/3 and 1/10 as potent as aspirin and indomethacin, respectively, in the acetic acid writhing assay in the rat.

b. Modified Trypsin Hyperalgesic Assay

This assay quantitatively measures analgesia and is designed to be unaffected by compounds possessing anti-inflammatory activity alone. The procedure described by Vinegar *et al. (Eur. J. Pharmacol., 37,* 23 (1976)) was modified so that drug administration preceded subplantar injection of trypsin (0.10 ml of a 0.10% solution of trypsin in pyrogen-free water) by 15 minutes.

In the modified trypsin hyperalgesic assay, Compound II has an $ED_{50} \simeq 50$ mg/kg p.o. Aspirin and indomethacin are inactive in this assay.

Example 11

Anti-Pyretic Activity

The Yeast-Induced Hyperthermia Assay was used according to the procedure described by Khalili-Varasteh *et al. (Arch. Int. Pharmacodyn, 219,* 149 (1976)) to demonstrate antipyretic activity.

Yeast-induced hyperthermia in the rat is completely reversed by 120 mg/kg p.o. of Compound II. Hypothermia was produced 3—4 hours after dosing. The $ED_{50}$ for aspirin in this assay is 50 mg/kg p.o. Aspirin does not produce hypothermia.

Example 12

Gastric Effects

a. Rat: Compound II does not produce gastric damage in the rat after a single oral dose of 150 mg/kg (4× the acute anti-inflammatory 3 hr. $ED_{50}$ and 15× the anti-platelet $ED_{50}$) or after 5 days of oral dosing at 75 mg/kg per day. A single oral dose of 30 mg/kg aspirin (anti-inflammatory 3 hr. $ED_{50}$) produces ulcers and hemorrhages, while a single oral dose of 5 mg/kg indomethacin ($2\frac{1}{2}$× the anti-inflammatory 3 hr. $ED_{50}$) produces ulcers.

b. *Guinea Pig:* At 100 mg/kg/day p.o. for 3 days, Compound II produces very slight gastric erosion in the guinea pig. This is in contrast to the severe ulceration and hemorrhage produced by the same dose of aspirin.

Example 13 Tablet

| | |
|---|---|
| Isethionate Salt of Compound II | 600 mg |
| Pregelatinized Corn Starch | 70 mg |
| Sodium Starch Glycolate | 26 mg |
| Magnesium Stearate | 4 mg |

The Compound II Isethionate was finely ground and intimately mixed with the powdered excipients, pregelatinized corn starch and sodium starch glycolate. The powders were wetted with purified water to form granules. The granules were dried and mixed with the magnesium stearate. The formulation was then compressed into tablets weighing approximately 700 mg each.

Example 14 Tablet

| | |
|---|---|
| Isethionate Salt of Compound II | 300 mg |
| Lactose | 212 mg |
| Corn Starch | 60 mg |
| Polyvinylpyrrolidone | 12 mg |
| Stearic Acid | 12 mg |
| Magnesium Stearate | 4 mg |

The Compound II Isethionate was finely ground and intimately mixed with the powdered excipients, lactose and corn starch. The powders were wetted with a solution of polyvinylpyrrolidone dissolved in purified water and denatured alcohol to form granules. The granules were dried and mixed with the powdered stearic acid and magnesium stearate. The formulation was then compressed into tablets weighing approximately 600 mg each.

Example 15 Capsule

| | |
|---|---|
| The Isethionate Salt of the Compound II | 300 mg |
| Lactose | 173 mg |
| Corn Starch | 55 mg |
| Stearic Acid | 22 mg |

The finely ground Compound II Isethionate was mixed with the powdered excipients lactose, corn starch, and stearic acid and filled into hard-shell gelatin capsules.

Example 16 Syrup

| | |
|---|---|
| Dihydrochloride of Compound II | 250 mg |
| Glycerin | 500 mg |
| Sucrose | 3,500 mg |
| Flavoring Agent | q.s. |
| Coloring Agent | q.s. |
| Preserving Agent | q.s. |
| Purified Water | q.s. to 5 ml |

The Compound II dihydrochloride and sucrose were dissolved in the glycerin and a portion of the purified water. The preserving agent was dissolved in another portion of hot purified water and then the coloring agent was added and dissolved. The two solutions were mixed and cooled before the flavoring agent was added. Purified water was added to final volume. The resulting syrup was thoroughly mixed.

Example 17 Injection

| | |
|---|---|
| Dihydrochloride Salt of Compound II | 100 mg |
| Sodium Chloride | 8.5 mg |
| Water for Injections | q.s. to 1.0 ml |

The finely ground active compound and sodium chloride were dissolved in the Water for Injections. The solution was filtered, filled into ampules and sterilized by autoclaving.

Example 18 Injection

| | |
|---|---|
| Dihydrochloride Salt of Compound II | 100 mg |
| Sodium Chloride | 8.5 mg |
| Preservative | q.s. |
| Water for Injection | q.s. to 1.0 ml |

The finely ground active compound, sodium chloride, and preservative were dissolved in the water for injection. The solution was sterilized by membrane filtration, aseptically filled into sterile multi-dose vials and sealed with sterile rubber stoppers.

Example 19 Tablet

| | |
|---|---|
| Monohydrochloride Salt of Compound II | 550 mg |
| Pregelatinized Corn Starch | 70 mg |
| Sodium Starch Glycolate | 26 mg |
| Magnesium Stearate | 4 mg |

The Compound II monohydrochloride was finely ground and intimately mixed with the powdered excipients, pregelatinized corn starch and sodium starch glycolate. The powders were wetted with purified water to form granules. The granules were dried and mixed with the magnesium stearate. The formulation was then compressed into tablets weighing approximately 650 mg each.

Example 20 Tablet

| | |
|---|---|
| Monohydrochloride Salt of Compound II | 275 mg |
| Lactose | 237 mg |
| Corn Starch | 60 mg |
| Polyvinylpyrrolidone | 12 mg |
| Stearic Acid | 12 mg |
| Magnesium Stearate | 4 mg |

The Compound II monohydrochloride was finely ground and intimately mixed with the powdered excipients, lactose and corn starch. The powders were wetted with a solution of polyvinylpyrrolidone dissolved in purified water and denatured alcohol to form granules. The granules were dried and mixed with the powdered stearic acid and magnesium stearate. The formulation was then compressed into tablets weighing approximately 600 mg each.

Example 21 Capsule

| | |
|---|---|
| Monohydrochloride Salt of Compound II | 275 mg |
| Lactose | 68 mg |
| Corn Starch | 40 mg |
| Sodium Lauryl Sulfate | 2 mg |
| Stearic Acid | 15 mg |

The finely ground Compound II monohydrochloride was mixed with the powdered excipients lactose, corn starch, sodium lauryl sulfate, and stearic acid and filled into hard-shell gelatin capsules.

Example 22 Tablet

| | |
|---|---|
| Compound II | 500 mg |
| Pregelatinized Corn Starch | 70 mg |
| Sodium Starch Glycolate | 26 mg |
| Magnesium Stearate | 4 mg |

The Compound II was finely ground and intimately mixed with the powdered excipients, pregelatinized corn starch and sodium starch glycolate. The powders were wetted with purified water to form granules. The granules were dried and mixed with the magnesium stearate. The formulation was then compressed into tablets weighing approximately 600 mg each.

Example 23 Tablet

| | |
|---|---|
| Compound II | 250 mg |
| Lactose | 219 mg |
| Corn Starch | 55 mg |
| Polyvinylpyrrolidone | 11 mg |
| Stearic Acid | 11 mg |
| Magnesium Stearate | 4 mg |

The Compound II was finely ground and intimately mixed with the powdered excipients, lactose and corn starch. The powders were wetted with a solution of polyvinylpyrrolidone dissolved in purified water and denatured alcohol to form granules. The granules were dried and mixed with the powdered stearic acid and magnesium stearate. The formulation was then compressed into tablets weighing approximately 550 mg each.

Example 24 Capsule

| | |
|---|---|
| Compound II | 250 mg |
| Lactose | 93 mg |
| Corn Starch | 40 mg |
| Sodium Lauryl Sulfate | 2 mg |
| Stearic acid | 15 mg |

The finely ground Compound II was mixed with the powdered excipients lactose, corn starch, sodium lauryl sulfate, and stearic acid and filled into hard-shell gelatin capsules.

**Claims**

1. Compounds of the general formula

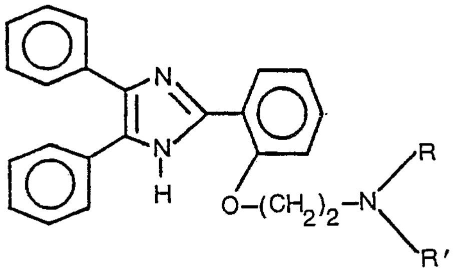

(I)

in which R and R′ are identical or different and may be hydrogen; a lower alkyl having 1 to 4 carbons; phenyl; optionally substituted with one or more lower alkyl groups having from 1 to 4 carbons, lower alkoxy groups having from 1 to 4 carbons or halogen atoms; phenyl-$C_{1-4}$-alkyl optionally substituted on the phenyl ring with one or more lower alkyl groups having from 1 to 4 carbons, lower alkoxy groups having 1 to 4 carbons or halogen atoms; or R and R′ taken together with the adjacent nitrogen atom form a cyclic substituent of 5 or 6 members containing in addition to the said nitrogen atom an optional second heteroatom selected from the group consisting of oxygen, nitrogen and sulphur) and acid addition salts and solvates thereof.

2. Compounds as claimed in claim 1 wherein at least one of R and R′ represents an alkyl group containing 1 or 2 carbon atoms.

3. Compounds as claimed in claim 1 or claim 2 wherein at least one of R and R′ represents a phenyl group optionally substituted by one or more radicals selected from methyl, ethyl, methoxy, ethoxy, bromo and chloro.

4. Compounds as claimed in any of claims 1 to 3 wherein at least one of R and R′ represents a phenyl-$C_{1-4}$ alkyl group optionally substituted on the phenyl ring and/or the alkylene group by one or more radicals selected from methyl, ethyl, methoxy, ethoxy, bromo and chloro.

5. Compounds as claimed in any of claims 1 to 4 wherein at least one of R and R′ represents a benzyl or phenylethyl group optionally substituted on the phenyl ring by one or more radicals selected from methyl, ethyl, methoxy, ethoxy, bromo and chloro.

6. Compound as claimed in claim 1 wherein R and R′ together with the adjacent nitrogen atom form a 5- or 6-membered ring optionally including a further heteroatom (in addition to the said nitrogen atom) selected from oxygen and nitrogen.

7. Compounds of formula (I) as claimed in any of the preceding claims in the form of their hydrochloride, sulfate, hydrogen sulfate, isethionate, maleate, mesylate, phosphate or tartrate acid addition salts.

8. 2-{2-[2-(Dimethylamino)ethoxy]phenyl}-4,5-diphenylimidazole and acid addition salts thereof.

9. 2-{2-[2-(Dimethylamino)ethoxy)phenyl}-4,5-diphenylimidazole monohydrochloride.

10. 2-{2-[2-(*N*-Methylbenzylamino)ethoxy]phenyl}-4,5-diphenylimidazole and acid addition salts thereof.

11. A compound of formula (I) (as defined in claim 1) selected from
2-[2-(2-aminoethoxy)phenyl]-4,5-diphenylimidazole
2-{2-[2-(methylamino)ethoxy]phenyl}-4,5-diphenylimidazole
2-{2-[2-(diethylamino)ethoxy]phenyl}-4,5-diphenylimidazole
2-{2-[2-(4-methoxy-*N*-methylbenzylamino)ethoxy]phenyl}-4,5-diphenylimidazole
2-{2-[2-(3,4-dimethoxy-*N*-methylbenzylamino)ethoxy]phenyl}-4,5-diphenylimidazole
2-{2-[2-(*N*,4-dimethylbenzylamino)ethoxy]phenyl}-4,5-diphenylimidazole
2-{2-[2-(4-chloro-*N*-methylbenzylamino)ethoxy]phenyl}-4,5-diphenylimidazole
4,5-diphenyl-2-{2-[2-(*N*-propylbenzylamino)ethoxy]phenyl}imidazole
2-{2-[2-(dibenzylamino)ethoxy]phenyl}-4,5-diphenylimidazole
2-{2-[2-(*N*-methylphenethylamino)ethoxy]phenyl}-4,5-diphenylimidazole
4,5-diphenyl-2-[2-(2-piperidinoethoxy)phenyl]imidazole
2-[2-(2-morpholinoethoxy)phenyl]-4,5-diphenylimidazole and
4,5-diphenyl-2-{2-[2-(1-pyrrolidinyl)ethoxy]phenyl}-imidazole,
and physiologically acceptable acid addition salts and solvates thereof.

12. A process for the preparation of a compound of formula (I) (as defined in claim 1) or an acid addition salt thereof which comprises

a) reacting an appropriate $\alpha,\beta$-diphenylethylene derivative with a 2-(2-aminoethoxy)phenyl compound to form a 2,4,5-trisubstituted imidazole ring and consequent formation of a compound of formula (I);

b) reacting a compound of formula

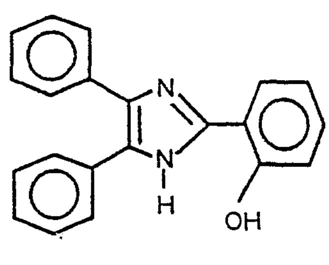

(X)

with a compound of formula

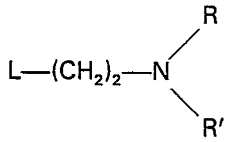

(XI)

(wherein R and R′ are as defined above and L is a leaving group);

c) reacting an amine of formula HNRR′ (wherein R and R′ are as defined above) with a compound of formula

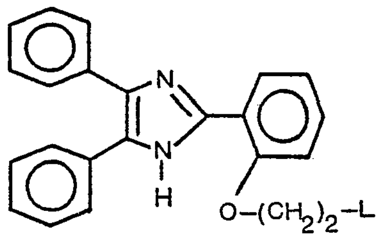

(XII)

(wherein L is a leaving group)

d) reacting a compound of formula (I) (wherein at least one of R and R′ is hydrogen) with an appropriate alkylating, arylating or aralkylating agent to provide a corresponding compound of formula (I) wherein at least one of R and R′ is a lower alkyl, phenyl or phenyl-$C_{1-4}$-alkyl group as defined above; or

e) condensing an amine of formula HNRR′ (wherein R and R′ are as defined above) with a compound of formula

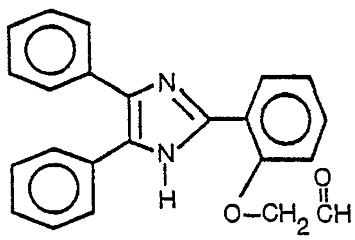

(XIV)

in the presence of a reducing agent; and optionally converting the resulting compound of formula (I) into an acid addition salt thereof.

13. Pharmaceutical formulations comprising, as active ingredient, at least one compound of formula

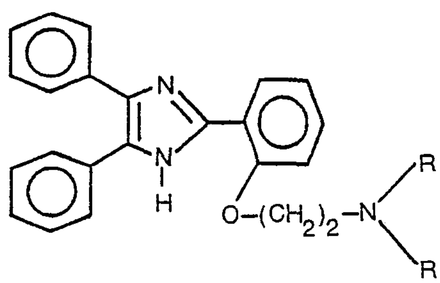

(I)

(in which R and R′ are identical or different and may be hydrogen; a lower alkyl having 1 to 4 carbons; phenyl optionally substituted with one or more lower alkyl groups having from 1 to 4 carbons, lower alkoxy groups having from 1 to 4 carbons or halogen atoms; phenyl-$C_{1-4}$-alkyl optionally substituted on the phenyl ring with one or more lower alkyl groups having from 1 to 4 carbons, lower alkoxy groups having 1 to 4 carbons or halogen atoms; or R and R′ taken together with the adjacent nitrogen atom form a cyclic substituent of 5 or 6 members containing in addition to the said nitrogen atom an optional second heteroatom selected from the group consisting of oxygen, nitrogen and sulphur) or a physiologically acceptable acid addition salt or solvate thereof, together with at least one pharmaceutical carrier or excipient.

14. Pharmaceutical formulations as claimed in claim 13 in dosage unit form.

15. Pharmaceutical formulations as claimed in claim 13 or claim 14 adapted for oral, rectal or parenteral administration.

16. Pharmaceutical formulations as claimed in any of claims 13 to 15 wherein the active

ingredient comprises 2-{2-[2-(dimethylamino)ethoxy]phenyl}-4,5-diphenylimidazole or a physiologically acceptable acid addition salt or solvate thereof.

17. Pharmaceutical formulations as claimed in any of claims 13 to 15 wherein the active ingredient comprises 2-{2-[2-(*N*-methylbenzylamino)ethoxy]phenyl}-4,5-diphenylimidazole or a physiologically acceptable acid addition salt or solvate thereof.

18. Pharmaceutical formulations as claimed in any of claims 13 to 15 wherein the active ingredient comprises at least one compound as claimed in claim 11.

19. Compounds of formula (I) and their physiologically acceptable acid addition salts and solvates for use in a method of treatment of the human or animal body by therapy.

20. Compounds of formula (I) and their physiologically acceptable acid addition salts for use in the treatment or prophylaxis of thrombo-embolic conditions in a mammal.

21. 2-{2-[2-(dimethylamino)ethoxy]phenyl}-4,5-diphenylimidazole or a physiologically acceptable acid addition salt or solvate thereof, for use in the treatment or prophylaxis of thrombo-embolic conditions in a mammal.

22. Compounds of formula (I) and their physiologically acceptable acid addition salts for use in the treatment or prophylaxis of inflammatory conditions in a mammal.

23. 2-{2-[2-(dimethylamino)ethoxy]phenyl}-4,5-diphenylimidazole or a physiologically acceptable acid addition salt or solvate thereof, for use in the treatment or prophylaxis of inflammatory conditions in a mammal.

24. Compounds of formula (I) and their physiologically acceptable acid addition salts for use as an antipyretic.

25. 2-{2-[2-(dimethylamino)ethoxy]phenyl}-4,5-diphenylimidazole or a physiologically acceptable acid addition salt or solvate thereof for use as an antipyretic.

26. Compounds of formula (I) and their physiologically acceptable acid addition salts for use as an analgesic.

27. 2-{2-[2-(dimethylamino)ethoxy]phenyl}-4,5-diphenylimidazole or a physiologically acceptable acid addition salt or solvate thereof for use as an analgesic.

**Patentansprüche**

1. Verbindungen der allgemeinen Formel I

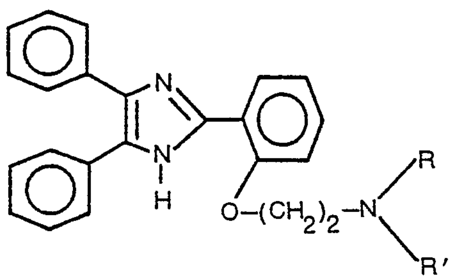

(I)

worin R und R′ gleich oder unterschiedlich sind und Wasserstoff; einen Niederalkylrest mit 1 bis 4 Kohlenstoffatomen; Phenyl, welches gegebenenfalls mit einem oder mehreren Niederalkylresten mit 1 bis 4 Kohlenstoffatomen, Niederalkoxyresten mit 1 bis 4 Kohlenstoffatomen oder Halogenatomen substituiert ist; Phenyl-$C_{1-4}$-alkyl, welches gegebenenfalls am Phenylring mit einer oder mehreren Niederalkylresten mit 1 bis 4 Kohlenstoffatomen, Niederalkoxyresten mit 1 bis 4 Kohlenstoffatomen oder Halogenatomen substituiert ist; bedeuten oder R und R′ zusammen mit dem benachbarten Stickstoffatom einen cyclischen Substituenten von 5 oder 6 Gliedern bilden, der zusätzlich zu dem Stickstoffatom ein wahlweises zweites Heteroatom enthält, welches unter Sauerstoff, Stickstoff und Schwefel ausgewählt ist, und Säureadditionssalze und Solvate davon.

2. Verbindungen nach Anspruch 1, worin mindestens einer der Reste R und R′ einen Alkylrest mit 1 bis 3 Kohlenstoffatomen darstellt.

3. Verbindungen nach Anspruch 1 oder Anspruch 2, worin zumindest einer der Reste R und R′ eine Phenylgruppe bedeutet, die gegebenenfalls durch einen oder mehrere Reste substituiert ist, welche unter Methyl, Ethyl, Methoxy, Ethoxy, Brom und Chlor ausgewählt sind.

4. Verbindungen nach einem der Ansprüche 1 bis 3, worin zumindest einer der Reste R und R′ einen Phenyl-$C_{1-4}$-alkylrest darstellt, der gegebenenfalls an dem Phenylring und/oder dem Alkylenrest durch ein oder mehrere Reste substituiert ist, die unter Methyl, Ethyl, Methoxy, Ethoxy, Brom und Chlor ausgewählt sind.

5. Verbindungen nach einem der Ansprüche 1 bis 4, worin zumindest einer der Reste R und R′ eine Benzyl- oder Phenylethylgruppe darstellt, die gegebenenfalls an dem Phenylring durch einen oder mehrere Reste substituiert ist, welche unter Methyl, Ethyl, Methoxy, Ethoxy, Brom und Chlor ausgewählt sind.

6. Verbindung nach Anspruch 1, worin R und R' zusammen mit dem benachbarten Stickstoffatom einen 5- oder 6-gliedrigen Ring bilden, der gegebenenfalls ein weiteres Heteroatom (zusätzlich zu dem genannten Stickstoffatom) einschließt, welches unter Sauerstoff und Stickstoff ausgewählt ist.

7. Verbindungen nach Formel I gemäß einem der voranstehenden Ansprüche in der Form ihrer Hydrochlorid-, Sulfat-, Hydrogensulfat-, Isothionat-, Maleat-, Mesylat-, Phosphat- oder Tartrat-Säureadditionssalze.

8. 2-{2-[2-(Dimethylamino)ethoxy]phenyl}-4,5-diphenylimidazol und dessen Säureadditionssalze.

9. 2-{2-[2-(Dimethylamino)ethoxy]phenyl}-4,5-diphenylimidazol-monohydrochlorid.

10. 2-{2-[2-(N-Methylbenzylamino)ethoxy]phenyl}-4,5-diphenylimidazol und dessen Säureadditionssalze.

11. Verbindung nach Formel I (gemäß der Definition in Anspruch 1), ausgewählt unter
2-[2-(2-Aminoethoxy)phenyl]-4,5-diphenylimidazol,
2-{2-[2-(Methylamino)ethoxy]phenyl}-4,5-diphenylimidazol,
2-{2-[2-(Diethylamino)ethoxy]phenyl}-4,5-diphenylimidazol,
2-{2-[2-(4-Methoxy-N-methylbenzylamino)ethoxy]phenyl}-4,5-diphenylimidazol,
2-{2-[2-(3,4-Dimethoxy-N-methylbenzylamino)ethoxy]phenyl}-4,5-diphenylimidazol,
2-{2-[2-(N-4-Dimethylbenzylamino)ethoxy]phenyl}-4,5-diphenylimidazol,
2-{2-[2-(4-Chlor-N-methylbenzylamino)ethoxy]phenyl}-4,5-diphenylimidazol,
4,5-Diphenyl-2-{2-[2-(N-propylbenzylamino)ethoxy]phenyl}-imidazol,
2-{2-[2-(Dibenzylamino)ethoxy]phenyl}-4,5-diphenylimidazol,
2-{2-[2-(N-Methylphenethylamino)ethoxy]phenyl}-4,5-diphenylimidazol,
4,5-Diphenyl-2-[2-(2-piperidinoethoxy)phenyl]imidazol,
2-[2-(2-Morpholinoethoxy)phenyl]-4,5-diphenylimidazol und
4,5-Diphenyl-2-{2-[2-(1-pyrrolidinyl)ethoxy]phenyl}-imidazol
und deren physiologisch verträgliche Säureadditionssalze und Solvate.

12. Verfahren zur Herstellung einer Verbindung nach Formel I (gemäß der Definition in Anspruch 1) oder eines Säureadditionssalzes davon, wobei

a) ein entsprechendes $\alpha,\beta$-Diphenylethylenderivat mit einer 2-(2-Aminoethoxy)phenylverbindung umgesetzt wird, um einen 2,4,5-trisubstituierten Imidazolring und anschließend eine Verbindung der Formel I zu bilden,

b) eine Verbindung der Formel X

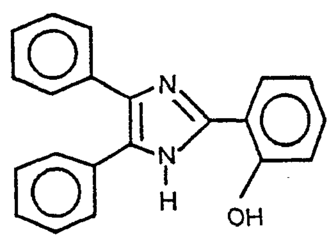

(X)

mit einer Verbindung der Formel

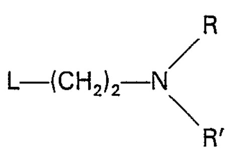

(XI)

(worin R und R' der oben genannten Definition entsprechen und L eine Abspaltgruppe ist) umgesetzt wird,

c) ein Amin der Formel HNRR' (worin R und R' der oben genannten Bedeutung entsprechen) mit einer Verbindung der Formel XII

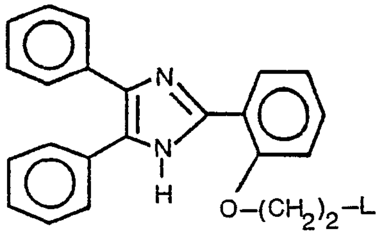

(XII)

(worin L eine Abspaltgruppe ist) umgesetzt wird,

d) eine Verbindung der Formel I (worin zumindest einer der Reste R und R' ein Wasserstoffatom ist) mit einem entsprechenden Alkylierungs-, Arylierungs- oder Aralkylierungsmittel umgesetzt wird, um eine entsprechende Verbindung der Formel I zur Verfügung zu stellen, worin zumindest einer der Reste R und R' ein Niederalkylrest, Phenyl oder Phenyl-$C_{1-4}$-alkylrest gemäß der obigen Definition ist, oder

e) ein Amin der Formel HNRR' (worin R und R' der oben genannten Definition entsprechen) mit einer Verbindung der Formel XIV

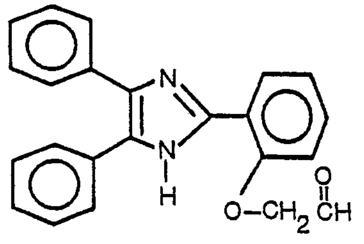

(XIV)

in Gegenwart eines Reduktionsmittels kondensiert wird, und gegebenenfalls die erhaltene Verbindung der Formel I in ein Säureadditionssalz davon überführt wird.

13. Pharmazeutische Formulierung, enthaltend als aktiven Bestandteil mindestens eine Verbindung der Formel I

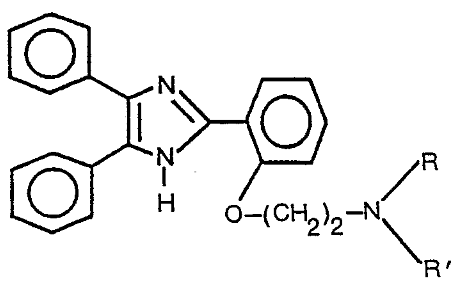

(I)

(worin R und R' gleich oder unterschiedlich sind und Wasserstoff; einen Niederalkylrest mit 1 bis 4 Kohlenstoffatomen; Phenyl, welches gegebenenfalls mit einem oder mehreren Niederalkylresten mit 1 bis 4 Kohlenstoffatomen, Niederalkoxyresten mit 1 bis 4 Kohlenstoffatomen oder Halogenatomen substituiert ist; Phenyl-$C_{1-4}$-alkyl, welches gegebenenfalls am Phenylring mit einem oder mehreren Niederalkylresten mit 1 bis 4 Kohlenstoffatomen, Niederalkoxyresten mit 1 bis 4 Kohlenstoffatomen oder Halogenatomen substituiert ist; bedeuten oder R und R' zusammen mit dem benachbarten Stickstoffatom einen cyclischen Substituenten von 5 oder 6 Gliedern bilden, der zusätzlich zu dem Stickstoffatom ein wahlweises zweites Heteroatom enthält, welches unter Sauerstoff, Stickstoff und Schwefel ausgewählt ist)
oder ein physiologisch verträgliches Säureadditionssalz oder Solvat davon, zusammen mit mindestens einem pharmazeutischen Träger oder Exzipienten.

14. Pharmazeutische Formulierungen nach Anspruch 13 in Dosierungseinheitsform.

15. Pharmazeutische Formulierungen nach Anspruch 13 oder Anspruch 14, die zur oralen, rectalen oder parenteralen Verabfolgung angepaßt sind.

16. Pharmazeutische Formulierungen nach einem der Ansprüche 13 bis 15, worin der aktive Bestandteil 2-{2-[2-(Dimethylamino)ethoxy]phenyl}-4,5-diphenylimidazol oder ein physiologisch verträgliches Säureadditionssalz oder ein Solvat davon enthält.

17. Pharmazeutische Formulierungen gemäß einem der Ansprüche 13 bis 15, worin der aktive Bestandteil 2-{2-[2-(N-Methylbenzylamino)ethoxy]phenyl}-4,5-diphenylimidazol oder eine physiologisch verträgliches Säureadditionssalz oder ein Solvat davon enthält.

18. Pharmazeutische Formulierungen nach einem der Ansprüche 13 bis 15, worin der aktive Bestandteil mindestens eine Verbindung gemäß Anspruch 11 enthält.

19. Verbindungen nach Formel I und deren physiologisch verträgliche Säureadditionssalze und Solvate zur Verwendung in einem therapeutischen Verfahren zur Behandlung des menschlichen oder tierischen Körpers.

20. Verbindungen nach Formel I und deren physiologisch verträgliche Säureadditionssalze zur Verwendung in der Behandlung oder Prophylaxe von thrombo-embolischen Zuständen bei einem Säugetier.

21. 2-{2-[2-(Dimethylamino)ethoxy]phenyl}-4,5-diphenylimidazol oder ein physiologisch verträgliches Säureadditionssalz oder Solvat davon zur Verwendung in der Behandlung oder Prophylaxe von thrombo-embolischen Zuständen bei einem Säugetier.

22. Verbindungen nach Formel I und deren physiologisch verträgliche Säureadditionssalze zur

Verwendung in der Behandlung oder Prophylaxe von Entzündungszuständen bei einem Säugetier.

23. 2-{2-[2-(Dimethylamino)ethoxy]phenyl}-4,5-diphenylimidazol oder ein physiologisch verträgliches Säureadditionssalz oder Solvat davon zur Verwendung in der Behandlung oder Prophylaxe von Entzündungszuständen bei einem Säugetier.

24. Verbindungen nach Formel I und deren physiologisch verträgliche Säureadditionssalze zur Verwendung als ein antipyretisches Mittel.

25. 2-{2-[2-(Dimethylamino)ethoxy]phenyl}-4,5-diphenylimidazol oder ein physiologisch verträgliches Säureadditionssalz oder Solvat davon zur Verwendung als ein antipyretisches Mittel.

26. Verbindungen nach Formel I und deren physiologisch verträgliche Säureadditionssalze zur Verwendung als ein Analgetikum.

27. 2-{2-[2-(Dimethylamino)ethoxy]phenyl}-4,5-diphenylimidazol oder ein physiologisch verträgliches Säureadditionssalz oder Solvat davon zur Verwendung als ein Analgetikum.

## Revendications

1. Composés de formule générale:

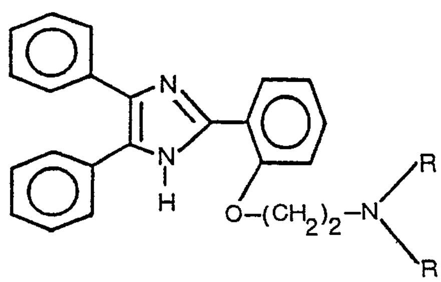

(I)

où R et R′ sont identiques ou différents et peuvent représenter un atome d'hydrogène; un radical alcoyle inférieur comptant 1 à 4 atomes de carbone; un radical phényle éventuellement substitué par un ou plusieurs radicaux alcoyle inférieurs comptant 1 à 4 atomes de carbone, radicaux alcoxy inférieurs comptant 1 à 4 atomes de carbone ou atomes d'halogène; un radical phényl-$C_{1-4}$-alcoyle éventuellement substitué sur le cycle phényle par un ou plusieurs radicaux alcoyle inférieurs comptant 1 à 4 atomes de carbone, radicaux alcoxy inférieurs comptant 1 à 4 atomes de carbone ou atomes d'halogène; ou R et R′ pris ensemble avec l'atome d'azote adjacent forment un substituant cyclique de 5 ou 6 chaînons comprenant en plus de l'atome d'azote un second hétéroatome facultatif choisi entre l'oxygène, l'azote et le soufre, et leurs sels d'addition d'acides et solvates.

2. Composés suivant la revendication 1, dans lesquels au moins l'un d'entre R et R′ représente un radical alcoyle comptant 1 ou 2 atomes de carbone.

3. Composés suivant la revendication 1 ou 2, dans lesquels au moins l'un d'entre R et R′ représente un radical phényle éventuellement substitué par un ou plusieurs radicaux choisis entre les radicaux méthyle, éthyle, méthoxy, éthoxy, bromo et chloro.

4. Composés suivant l'une quelconque des revendications 1 à 3, dans lesquels au moins l'un d'entre R et R′ représente un radical phényl-$C_{1-4}$-alcoyle éventuellement substitué sur le cycle phényle et/ou le radical alcoylène par un ou plusieurs radicaux choisis entre les radicaux méthyle, éthyle, méthoxy, éthoxy, bromo et chloro.

5. Composés suivant l'une quelconque des revendications 1 à 4, dans lesquels au moins l'un d'entre R et R′ représente un radical benzyle ou phényléthyle éventuellement substitué sur le cycle phényle par un ou plusieurs radicaux choisis entre les radicaux méthyle, éthyle, méthoxy, éthoxy, bromo et chloro.

6. Composé suivant la revendication 1, dans lequel, R et R′ forment avec l'atome d'azote adjacent un cycle à 5 ou 6 chaînons comprenant éventuellement un hétéroatome supplémentaire (en plus de cet atome d'azote) choisi entre l'oxygène et l'azote.

7. Composés de formule (I) suivant l'une quelconque des revendications précédentes sous la forme de leurs chlorhydrates, sulfates, hydrogénosulfates, iséthionates, maléates, mésylates, phosphates ou tartrates d'addition d'acides.

8. Le 2-{2-[2-(diméthylamino)éthoxy]phényl}-4,5-diphénylimidazole) et ses sels d'addition d'acides.

9. Le monochlorhydrate du 2-{2-[2-(diméthylamino)éthoxy]phényl}-4,5-diphénylimidazole.

10. Le 2-{2-[2-(*N*-méthylbenzylamino)éthoxy]phényl}-4,5-diphénylimidazole et ses sels d'addition d'acides.

11. Composé de formule (tel que défini dans la revendication 1) choisi parmi:

le 2-[2-(2-aminoéthoxy)phényl]-4,5-diphénylimidazole
le 2-{2-[2-(méthylamino)éthoxy]phényl}-4,5-diphénylimidazole
le 2-{2-[2-(diéthylamino)éthoxy]phényl}-4,5-diphénylimidazole
le 2-{2-[2-(4-méthoxy-*N*-méthylbenzylamino)éthoxy]phényl}-4,5-diphénylimidazole
le 2-{2-[2-(3,4-diméthoxy-*N*-méthylbenzylamino)éthoxy]phényl}-4,5-diphénylimidazole
le 2-{2-[2-(*N*,4-diméthylbenzylamino)éthoxy]phényl}-4,5-diphénylimidazole
le 2-{2-[2-(4-chloro-*N*-méthylbenzylamino)éthoxy]phényl}-4,5-diphénylimidazole
le 4,5-diphényl-2-{2-[2-(*N*-propylbenzylamino)éthoxy]phényl}imidazole
le 2-{2-[2-(dibenzylamino)éthoxy]phényl}-4,5-diphénylimidazole
le 2-{2-[2-(*N*-méthylphénéthylamino)éthoxy]phényl}-4,5-diphénylimidazole
le 4,5-diphényl-2-[2-(2-pipéridinoéthoxy)phényl]imidazole
le 2-[2-(2-morpholinoéthoxy)phényl]-4,5-diphénylimidazole, et
le 4,5-Diphényl-2-{2-[2-(1-pyrrolidinyl)éthoxy]phényl}imidazole
et leurs sels d'addition d'acides et solvates physiologiquement acceptables.

12. Procédé de préparation d'un composé de formule (I) (tel que défini dans la revendication 1) ou d'un sel d'addition d'acide de celui-ci, qui comprend

a) la réaction d'un dérivé d'$\alpha,\beta$-diphényléthylène approprié avec un dérivé de 2-(2-aminoéthoxy)phényle pour la formation d'un cycle d'imidazole 2,4,5-trisubstitué et ainsi la formation d'un composé de formule (I):

b) la réaction d'un composé de formule:

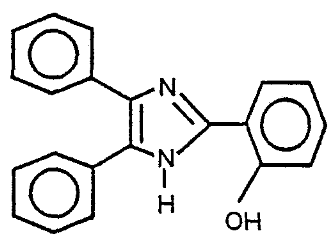

(X)

avec un composé de formule

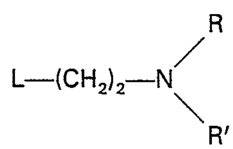

(XI)

(où R et R' sont tels que définis ci-dessus et L représente un radical labile);

c) la réaction d'une amine de formule HNRR' (où R et R' sont tels que définis ci-dessus) avec un composé de formule:

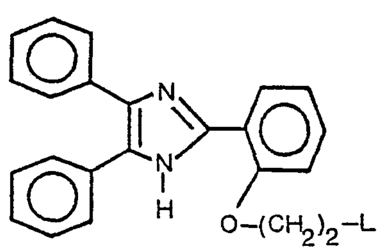

(XII)

(où L représente un radical labile)

d) la réaction d'un composé de formule (I) (où au moins l'un d'entre R et R' représente un atome d'hydrogène) avec un agent d'alcoylation, d'arylation ou d'aralcoylation approprié pour la formation d'un composé correspondant de formule (I) dans lequel au moins l'un d'entre R et R' représente un radical alcoyle inférieur, phényle ou phényl-$C_{1-4}$-alcoyle tel que défini ci-dessus, ou

e) la condensation d'une amine de formule HNRR' (où R et R' sont tels que définis ci-dessus) avec un composé de formule:

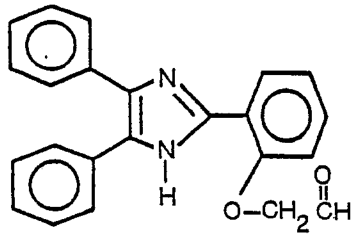

(XIV)

en présence d'un réducteur,
et éventuellement la conversion du composé résultant de formule (I) en un sel d'addition d'acide de celui-ci.

13. Compositions pharmaceutiques comprenant, comme principe actif, au moins un composé de formule

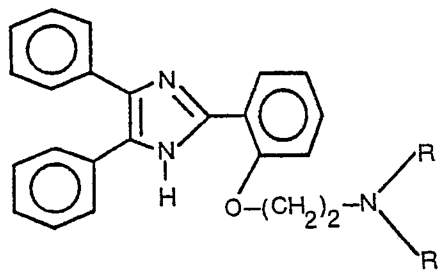

(I)

où R et R′ sont identiques ou différents et peuvent représenter un atome d'hydrogène; un radical alcoyle inférieur comptant 1 à 4 atomes de carbone; un radical phényle éventuellement substitué par un ou plusieurs radicaux alcoyle inférieurs comptant 1 à 4 atomes de carbone, radicaux alcoxy inférieurs comptant 1 à 4 atomes de carbone ou atomes d'halogène; un radical phényl-$C_{1-4}$-alcoyle éventuellement substitué sur le cycle phényle par un ou plusieurs radicaux alcoyle inférieurs comptant 1 à 4 atomes de carbone, radicaux alcoxy inférieurs comptant 1 à 4 atomes de carbone ou atomes d'halogène; ou R et R′ pris ensemble avec l'atome d'azote adjacente forment un substituant cyclique de 5 ou 6 chaînons comprenant en plus de l'atome d'azote un second hétéroatome facultatif choisi entre l'oxygène, l'azote et le soufre,
ou un sel d'addition d'acide ou solvate physiologiquement acceptable de celui-ci, conjointement avec au moins un excipient ou diluant pharmaceutique.

14. Compositions pharmaceutiques suivant la revendication 13, sous forme dosées unitaires.

15. Compositions pharmaceutiques suivant la revendication 13 ou 14, propres à l'administration par voie orale, rectale ou parentérale.

16. Compositions pharmaceutiques suivant l'une quelconque des revendications 13 à 15, dans lesquelles le principe actif comprend du 2-{2-[2-(diméthylamino)éthoxy]phényl}-4,5-diphénylimidazole) ou un sel d'addition d'acide ou solvate physiologiquement acceptable de celui-ci.

17. Compositions pharmaceutiques suivant l'une quelconque des revendications 13 à 15, dans lesquelles le principe actif comprend du 2-{2-[2-(*N*-méthylbenzylamino)éthoxy]phényl}-4,5-diphénylimidazole ou un sel d'addition d'acide ou solvate physiologiquement acceptable de celui-ci.

18. Compositions pharmaceutiques suivant l'une quelconque des revendications 13 à 15, dans lesquelles le principe actif comprend au moins un composé suivant la revendication 11.

19. Composés de formule (I) et leurs sels d'addition d'acides et solvates physiologiquement acceptables à utiliser pour le traitement thérapeutique somatique d'un être humain ou d'un animal.

20. Composés de formule (I) et leurs sels d'addition d'acides physiologiquement acceptables à utiliser pour le traitement ou la prophylaxie d'états thromboemboliques chez un mammifère.

21. Le 2-{2-[2-(diméthylamino)éthoxy]phényl}-4,5-diphénylimidazole ou un sel d'addition d'acide ou solvate physiologiquement acceptable de celui-di à utiliser pour le traitement ou la prophylaxie d'états thromboemboliques chez un mammifère.

22. Composés de formule (I) et leurs sels d'addition d'acides physiologiquement acceptables à utiliser pour le traitement ou la prophylaxie d'états inflammatoires chez un mammifère.

23. Le 2-{2-[2-(diméthylamino)éthoxy]phényl}-4,5-diphénylimidazole ou un sel d'addition d'acide ou solvate physiologiquement acceptable de celui-ci à utiliser pour le traitement ou la prophylaxie d'états inflammatoires chez un mammifère.

24. Composés de formule (I) et leurs sels d'addition d'acides physiologiquement acceptables à utiliser comme antipyrétiques.

25. Le 2-{2-[2-(diméthylamino)éthoxy]phényl}-4,5-diphénylimidazole ou un sel d'addition d'acide ou solvate physiologiquement acceptable de celui-ci à utiliser comme antipyrétique.

26. Composés de formule (I) et leurs sels d'addition d'acides physiologiquement acceptables à utiliser comme analgésiques.

27. Le 2-{2-[2-(diméthylamino)éthoxy]phényl}-4,5-diphénylimidazole ou un sel d'addition d'acide ou solvate physiologiquement acceptable de celui-ci à utiliser comme analgésique.